# EUROPEAN PATENT SPECIFICATION

(11) **EP 4 182 522 B1**
(45) Date of publication and mention of the grant of the patent: **21.10.2026**
(21) Application number: 21843406.6
(22) Date of filing: 14.07.2021
(51) Int. Cl.: E05B 1/00, A61L 2/00, A61L 2/10, A61L 2/24

(54) **DOOR HANDLE SANITIZING DEVICE**
TÜRGRIFFDESINFEKTIONSVORRICHTUNG
DISPOSITIF DE DÉSINFECTION DE POIGNÉE DE PORTE

(30) Priority: 17.07.2020 US 202016932221
(43) Date of publication of application: 24.05.2023
(73) Proprietor: Germ Dome Industries LLC, Orchard Lake, MI 48324 (US)
(72) Inventor: SCHUMACHER, Dean, Bloomfield, Michigan 48324 (US)
(74) Representative: Forresters IP LLP
(86) International application number: PCT/US2021/041606
(87) International publication number: WO 2022/015837

(56) References cited:
- WO-A2-2009/056765
- CN-U- 202 672 848
- DE-A1- 102008 063 887
- KR-A- 20200 016 717
- KR-Y1- 200 410 373
- US-A1- 2011 174 992
- US-A1- 2018 085 481
- US-B1- 7 989 779
- US-B2- 10 092 669

## Description

### BACKGROUND

### a. Technical Field

The present disclosure relates to a device for sanitizing a point of contact. More specifically, this disclosure relates to a system and apparatus for sanitizing a door handle, door knob, or similar structure or device.

### b. Background Art

Door handles, door knobs, other door opening devices, and other points of contact for large groups of people, such as automatic teller machines (ATMs) and elevator buttons or control panels, can be involved in the spread of communicable germs. In hospitals, germs communicated through points of contact can lead to increased patient sickness. In an office environment, germs communicated through points of contact can lead to sick employees and lost productivity. In public spaces, germs communicated through points of contact can lead to a sick populace and spread of disease.

The effect of germs spread through points of contact can be significantly reduced by sanitizing the surface of the point of contact periodically, such as after each use of the point of contact. There is therefore a need for a device to sanitize point-of-contact surfaces to eliminate one or more of the problems described above.
US20110174992 discloses a door handle irradiation system including at least one local subsystem that includes at least one door handle irradiation hardware, at least one motion detectors, at least one door open/close state sensor; and at least one device to program the local subsystem. The device to program the local subsystem is connected to the local subsystem by a wired or wireless communication means. The local subsystem includes a control means, door handle irradiation hardware, at least one motion detector and at least one door open/closed state sensor. The door handle irradiation hardware includes a housing that contains a plurality of germicidal lamps. The housing is generally a letter C- or reverse letter C-shaped, and comprises a vertical part and two horizontal parts, contiguous to one another, and each part contains at least one germicidal lamp that emits ultraviolet rays.
US7989779 discloses a universal ultraviolet light sanitation device that can be mounted to existing doors and the like wherein the UV light sources are positioned for treatment of a door knob or handle. The device provides for opposing UV lights in a light emitting housing around the doorknob. Sensor means within the assembly determines the non-presence of the user's hand and activates the UV lights before and after handle contact assuring sanitation of the light exposed non-porous surfaces.
US20180085481 discloses an apparatus for sanitizing a point-of-contact surface may include a housing configured to be affixed about at least a portion of said point of contact. The housing may have an interior in or through which the point of contact may be accessed and an anterior opening for access to the interior. The apparatus may further include one or more sources of a sanitizing agent, such as germicidal light, configured to direct the sanitizing agent toward a location where at least a portion of the point-of-contact surface may be disposed. One or more of an anterior dome, an inner surface, and a posterior surface may also be configured to reflect the sanitizing agent toward the location where at least a portion of the point-of-contact surface may be disposed.
KR20200016717 discloses a device to effectively disinfect germs transferred from hand to hand in public facilities. An internal iron piece is installed near the middle of a door. An ultraviolet lamp is installed on the internal iron piece. A piezoelectric element is installed in a joint between the internal iron piece and the door. A protective cover enables cleaning without touching the ultraviolet lamp directly.
Aspects of the present invention are recited by the independent claims, with the dependent claims reciting optional features.

### BRIEF DESCRIPTION OF THE DRAWINGS

Figure 1 is a perspective view of an example door handle sanitizing device coupled to a door adjacent to an example door handle.
Figure 2 is an upper perspective view of an example door handle sanitizing device.
Figure 3 is a lower perspective view of the example door handle sanitizing device of Figure 2.
Figure 4 is a left-side view of the example door handle sanitizing device of Figure 2, the right-side being a mirror image.
Figure 5 is a bottom view of the example door handle sanitizing device of Figure 2.
Figure 6 is a rear view of the example door handle sanitizing device of Figure 2, with the mounting plate omitted to illustrate the back of the body of the device.
Figure 7 is a side view of the example door handle sanitizing device, illustrating the projection field of the sanitizing agent of the device.
Figure 8 is an exploded view of the example door handle sanitizing device of Figure 2.
Figure 9 is a block diagram of a portion of the example door handle sanitizing device of Figure 2.

### DETAILED DESCRIPTION OF THE INVENTION

It is known in the art that certain sanitizing agents may be used to sanitize objects and surfaces, such as door handles, door knobs, other door opening devices, automatic teller machines (ATMs), or elevator buttons and control panels. One type of sanitizing agent is electromagnetic energy of a particular frequency in the ultraviolet (UV) light spectrum. Ultraviolet light with a wavelength in the range of about 100 nanometers to about 280 nanometers (referred to herein as germicidal UV) is known to be deadly to bacteria and other micro-organisms, killing the micro-organisms, rendering them harmless, or eliminating their ability to reproduce. Germicidal UV with a wavelength of about 254 nanometers (a frequency of about 1.18 petahertz (PHz)) is known to be particularly effective. In one embodiment of the present invention, germicidal UV may be used as an effective agent for sanitizing doorknobs, other door opening devices, automatic teller machines (ATMs), elevator buttons and control panels, and other devices and apparatus that may be points of contact for a large volume of people, referred to herein as "points of contact." Similarly, as used herein, a "point-of-contact surface" refers to any surface of a point of contact. A device for sanitizing such point-of-contact surfaces may involve germicidal UV or other sanitizing agents.

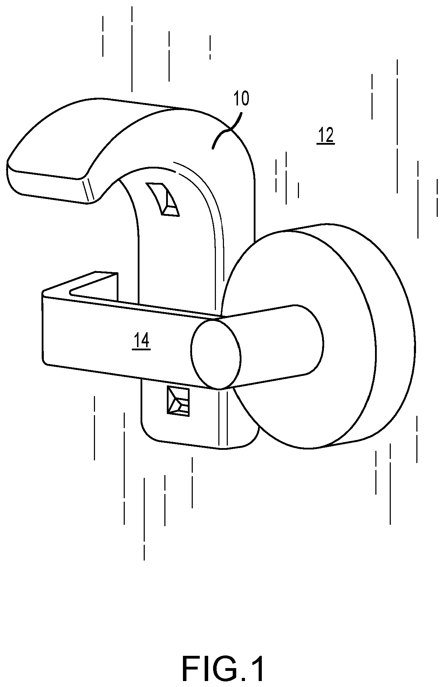

Referring now to the drawings wherein like reference numerals are used to identify identical components in the various views, FIG. 1 is a perspective view of an example door handle sanitizing device 10 disposed on an example door 12 adjacent to an example door handle 14 so as to sanitize the door handle 14.

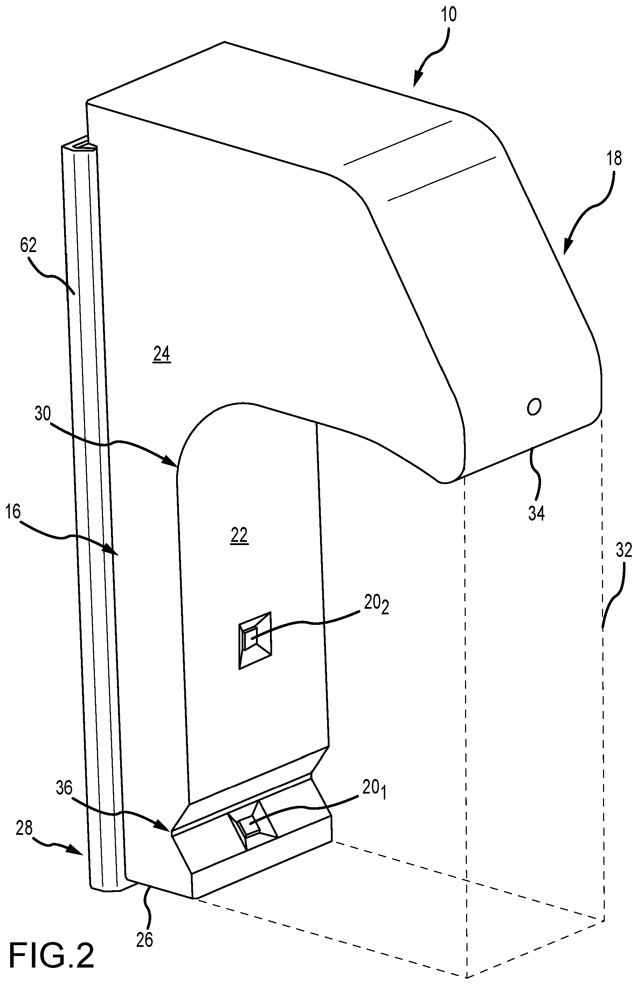

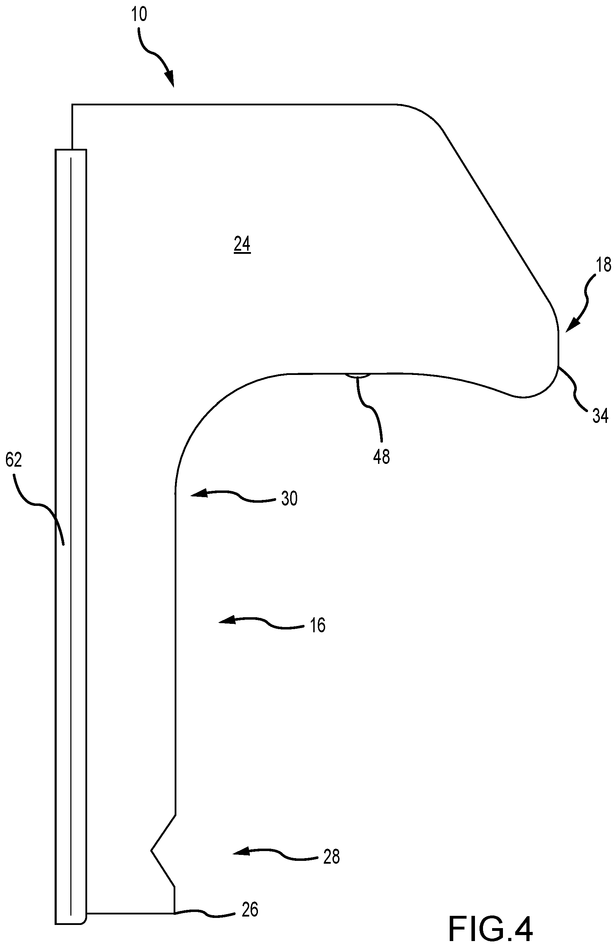

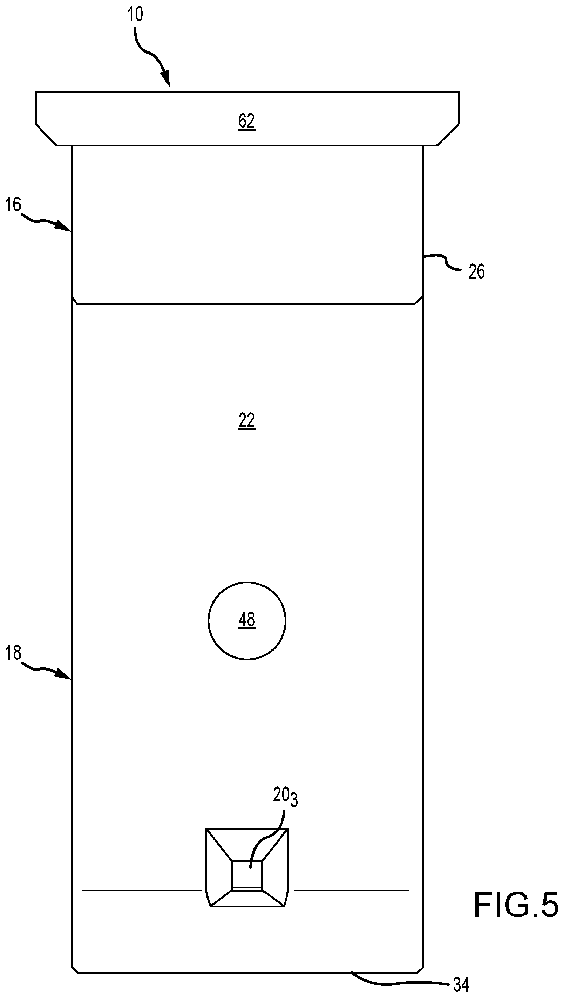

FIGS. 2-5 are various views of the door handle sanitizing device 10 (which may be referred to herein as "the device 10" or "the apparatus 10") in isolation. Referring to FIGS. 1-6, the device 10 includes a linear portion 16 defining a posterior wall, an arched or curved portion 18, and one or more sources of a sanitizing agent 20 disposed on the linear portion 16 and/or arched portion 18 so as to sanitize the door handle 14 or other point of contact disposed adjacent to the device 10.

The linear portion 16 and arched portion 18 may share a common inner surface 22 and opposed sidewalls 24. The sidewalls 24 may be generally parallel to one another and generally transverse to the door 12, in some configurations (such as the configuration shown in FIG. 1). In other configurations, the device 10 may be coupled with a door 12 such that the sidewalls 24 are parallel with the door 12 and the inner surface 22 is transverse to the door 12.

The linear portion of the inner surface 22 may extend from a linear tip 26 at a bottom portion 28 of the device 10 to a transition portion 30. The arched portion 18 may extend in an anterior direction from the transition portion 30. The transition portion 30 may be at a top of the linear portion 16. The arched 18 portion includes a concave curve. Accordingly, in an embodiment, the inner surface 22 may form an approximately J-shaped surface (e.g., the transverse cross-section of the inner surface 22 may form a J-shape).

The inner surface 22 may define a projected three-dimensional volume 32 (shown in FIG. 2) that is bounded on its anterior side by an anterior-most point 34 of the arched portion 18 (and a downward plane from the anterior-most point 34 that is parallel with inner surface 22 at linear portion 16), on its upper side by the arched portion 18, on its lateral ends by planes normal to the lateral edges of the inner surface 22, and on its lower side by a normal plane from the linear tip 26. Accordingly, the volume 32 may extends anterior from the inner surface 22 of the linear portion 16, with the bottom of the volume 32 extending anterior from the linear tip 26. The device 10 may be positioned relative to a door handle 14 or other point of contact such that the door handle is disposed within or extends through the three-dimensional volume 32. Within the volume 32 may be projected two-dimensional areas (e.g., planes) that are bounded by the inner surface 22 on the posterior and upper sides and by normal planes from the linear tip 26 and anterior-most point 34. The device 10 may be positioned relative to a door handle 14 or other point of contact such that the door handle extends through one or more such two-dimensional planes. In some embodiments, the volume 32 may have a height of between 8 cm (three inches) and 23 cm (nine inches), a width of between 1 cm (a half inch) and 8 cm (three inches), and a depth (from anterior to posterior) of between 3 cm (one inch) and 15 cm (six inches), for example.

The example device 10 includes three sanitizing agent sources 20₁, 20₂, 20₃, which may be referred to collectively as sources 20 or individually as a source 20. More or fewer sanitizing agent sources 20 may be provided in different embodiments (e.g., two sources 20, four sources 20, and so on).

One or more of the sources 20 may be a source of ultraviolet (UV) light, such as germicidal UV. In some embodiments, one or more of the sources 20 may be one or more germicidal UV LEDs. In some embodiments, all of the sources 20 may be germicidal UV LEDs.

A first source 20₁ may be disposed at a bottom portion 28 of the device and on the linear portion 16. The first source 20₁ may be angled upward so as to project the sanitizing agent (e.g., germicidal UV light) in an anterior direction towards the arched portion 18. In some embodiments, the first source 20₁ may be angled at between 15 and 75 degrees with respect to the primary plane of the inner surface 22 (and, thus, with respect to the door 12 when the device 10 is coupled to the door in the manner illustrated in FIG. 1). In some embodiments, the first source 20₁ may be angled at between 30 and 60 degrees *(i.e.,* configured to project light at such an angle) with respect to the primary plane of the inner surface 22 (that is, the primary plane within the linear portion 16). In some embodiments, the first source 20₁ may be angled at about 45 degrees with respect to the primary plane of the inner surface 22. As used herein, the numerical values of angles of light projection of a source 20 refers to the center of the projection field of the source 20.

The linear portion 16 (e.g., the inner surface 22 thereof) may include a recess or notch 36 within which the first source 20₁ may be disposed. The recess 36 may span the entire width of the inner surface 22 *(e.g.,* from a first sidewall 24 to the opposite sidewall 24), in some embodiments, so as to provide the maximum light projection by the first source 20₁ from within the recess 36. The first source 20₁ may be disposed on a surface of the recess 36 having one of the above-noted angles with respect to the primary plane of the inner surface 22.

The third source 20₂ may be disposed proximate a midpoint (e.g., between the linear tip 26 and the transition portion 30) of the linear portion 16, on the inner surface 22. The third source 20₂ may be configured to project light in an anterior direction at a 90-degree angle with respect to the primary plane of the inner surface 22 (*i.e.,* normal to the primary plane).

The second source 20₃ may be disposed on the inner surface 22 of the arched portion 18. The second source 20₃ may be configured to project light in a posterior direction towards the linear portion 16. That is, the second source may be angled to project sanitizing agent towards the door 12 when the device 10 is coupled to the door 12 as shown in FIG. 1.

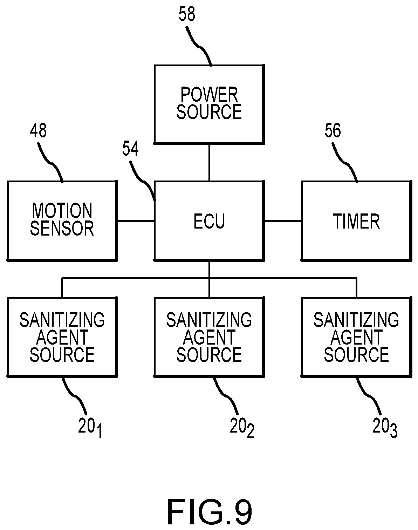

The device 10 may further include a motion sensor 48 disposed on the inner surface 22 of the arched portion 18. The motion sensor 48 may thus be disposed under the arch of the arched portion 18. The motion sensor 48 may be configured to detect nearby motion, in response to which the sources 20 may be controlled, as will be described with respect to FIG. 9.

The motion sensor 48 may be positioned on the device 10 and have a desired sensitivity so as to detect motion within a desired distance of the device 10 (or within a desired distance of the point of contact sanitized by the device 10). In some embodiments, the motion sensor 48 may detect motion within 3 m (10 feet) or less, 2.4 m (8 feet) or less, 1.5 m (5 feet) or less, 1 m (3 feet) or less, 0.3 m (1 foot) or less, or within another desired distance from the device 10. In an embodiment, the sensitivity of the motion sensor 48 may be selected according to the power of the sources 20. For example, in an embodiment in which the sources 20 are each germicidal UV LEDs having respective output intensities of two millijoules, the motion sensor 48 may be configured to detect motion within 0.3 m (1 foot) of the device. In some embodiments, the effective range of the motion sensor 48 may be implemented in processing of the output of the motion sensor 48.

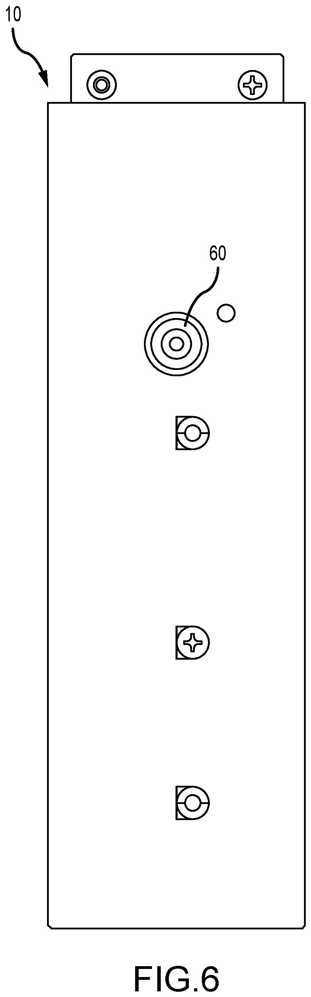

FIG. 6 is a posterior view of the device 10, with the mounting bracket 62 omitted to show the back of the device 10. As shown in FIG. 6, the device 10 may include an electrical port 60 configured to receive a power input that may directly power the device 10 and/or charge a battery of the device 10, for example.

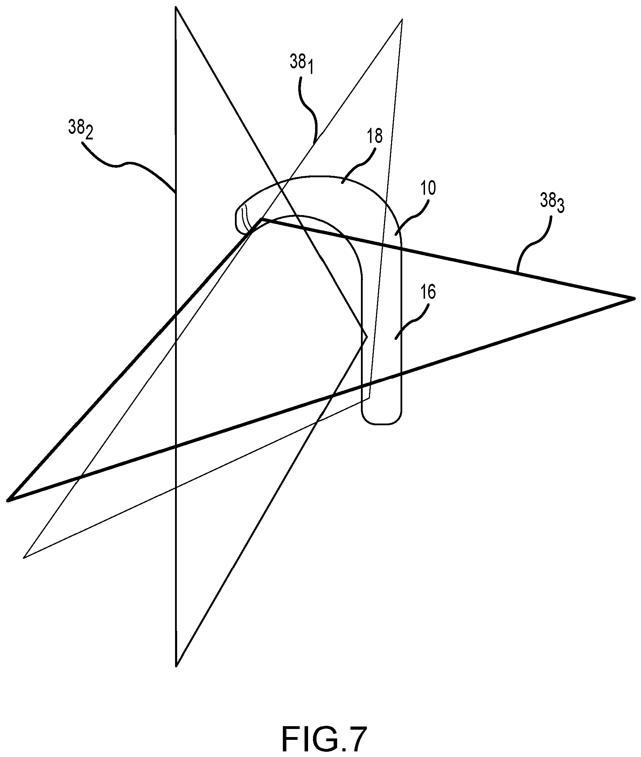

FIG. 7 is a diagrammatic side view illustrating example light projection fields and angles of the three sources 20₁, 20₂, 20₃. Each source 20₁, 20₂, 20₃ may have a respective projection field 38₁, 38₂, 38₃ (which may be referred to individually as a projection field 38 or collectively as projection fields 38). One or more of the projection fields *(e.g.,* each projection field) 38 may have a width and/or height of between 90 degrees and 150 degrees, in some embodiments. One or more of the projection fields *(e.g.,* each projection field) 38 may have a width and/or height of between 100 degrees and 140 degrees, in some embodiments. One or more of the projection fields *(e.g.,* each projection field) 38 may have a width and/or height of between 110 degrees and 130 degrees, in some embodiments. One or more of the projection fields *(e.g.,* each projection field) 38 may have a width and/or height of about 120 degrees, in some embodiments.

As illustrated in FIG. 7, the sources 20₁, 20₂, 20₃ may be arranged and configured so as to have overlapping fields 38 and/or to project sanitizing agent towards one another. For example, the field 38₁ of the first source 20₁ and the field 38₂ of the third source 20₂ may include the arched portion 18, and the field 38₃ of the second source 20₃ may include the linear portion 16. Further, the sources 20 may be arranged so that the fields 38 envelope the door handle 12 or other point of contact by striking the point of contact from different positions and angles.

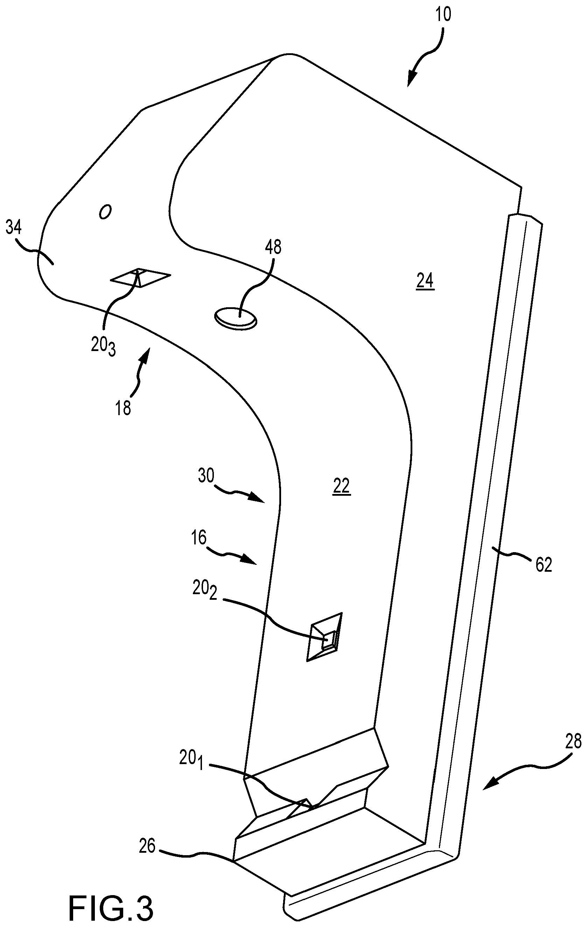

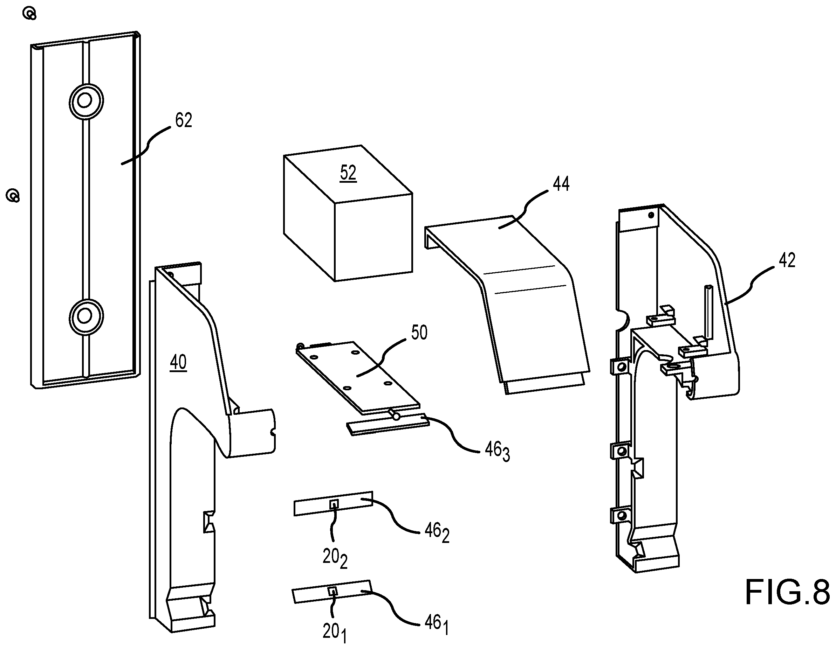

FIG. 8 is an exploded view of the device 10 illustrating an example construction of the device 10. The device 10 may include two lateral body portions 40, 42 and a cover 44 collectively defining the body of the device 10 and defining apertures through which the sources 20₁, 20₂, 20₃ may project a sanitizing agent, such as germicidal ultraviolet light. The device may further include three LED chips 46₁, 46₂, 46₃ on which the sources 20₁, 20₂, 20₃ are respectively disposed, and a motion sensor chip 50 on which the motion sensor 48 (not designated in FIG. 8; designated in FIGS. 3, 4, and 5) is disposed. An electronics unit 52 may be provided that includes a power source, such as a battery, and an electronic control unit, which may comprise a combined processor and memory in some embodiments, for controlling the sources 20 in response to various stimuli (e.g., detected motion) and/or control strategies. The device 10 may further include a mounting bracket 62, which may be removably coupled from the body of the device 10 and which may be affixed to a door, wall, or other structure adjacent to a point of contact intended to be sanitized by the device 10. The mounting bracket 62 is also shown in FIGS. 2-5, but omitted in FIGS. 1, 6, and 7.

FIG. 9 is a block diagram of a portion of the device 10. The device portion may include an electronic control unit (ECU) 54 in electronic communication with the sources 20₁, 20₂, 20₃, the motion sensor 48, a timer 56, and a power source 58. The ECU 54, sources 20, motion sensor 48, timer 56, and power source 58 may all be disposed in or on a body of the device 10, in some embodiments. The ECU 54 may include a non-transitory computer-readable memory storing instructions and a processor configured to execute the instructions on the memory to perform one or more operations or functions of the ECU 54. The timer 56 may be configured to track a time of day, a time elapsed since a previous sanitization or detected motion, or the time elapsed or remaining in a current sanitization cycle. In some embodiments, the timer 56 may be functionality of the ECU 54. The power source 58 may be one or more batteries, in some embodiments, that may be removable, replaceable, and/or rechargeable. In other embodiments, the power source may be a power supply configured to receive AC or DC input power from a persistent source, such as a wall outlet or other mains power supply.

The ECU 54 may be configured to activate or deactivate one or more sources 20 by instructing one or more of the sources 20 to activate or deactivate, by selectively providing power from the power source 58 to one or more of the sources 20, or otherwise. In some embodiments, the ECU 54 may be configured to activate and deactivate one or more sources 20 to effect a sanitization cycle of a predefined duration, such as 2 seconds, 3 seconds, 5 seconds, 10 seconds, 20 seconds, 30 seconds, 60 seconds, or another appropriate duration for sanitizing the door handle or other point of contact, during which sanitization cycle the sources 20 may output the sanitizing agent. The ECU 54 may be configured to execute a sanitization cycle on a predefined schedule, such as every 10 minutes, every 30 minutes, or every hour, in some embodiments. The sanitization cycle may be programmed depending on the time of day, in some embodiments, such as to be more frequent during business hours, for example. In some embodiment, the ECU 54 may receive an indication from the motion sensor 48 that motion was sensed and control a sanitization cycle responsive to the detected motion. For example, the ECU 54 may be configured to begin a sanitization cycle a set period of time after motion is sensed, such as 3 seconds, 5 seconds, 10 seconds, 15 seconds, or another period after motion was last detected. In another example, the ECU 54 may be configured to pause a sanitization cycle when motion is detected (e.g., so as to avoid exposing a user's hand, a nearby child's eyes, or other sensitive body part to germicidal UV or other sanitizing agent), and resume the sanitization cycle or beginning a new sanitization cycle a set period of time after the last motion was sensed, such as 3 seconds, 5 seconds, 10 seconds, 15 seconds, or another period of time. In some embodiments, a sanitization cycle may be executed in discrete segments, such as a first cycle segment of one of the above-noted periods of time, a pause of a predetermined duration, and a second cycle segment of one or of above-noted periods of time.

In some embodiments, in addition to or instead of a timer 56 and/or a motion sensor 48, the device 10 may include a manual switch that may be connected to the ECU 54 to activate or deactivate a sanitization cycle. Such a switch may be disposed on a body of the device 10 or adjacent to the body, for example, and may be in wired or wireless communication with the ECU 54.

A device for sanitizing a point-of-contact surface, such as, for example, a door handle, other door opening device, ATM, or elevator buttons or control panel, as described herein has many advantages. The reduction of communicable germs in hospitals, where individuals may have weakened immune systems, may reduce loss of life and other illness resulting from transmitted germs. The reduction of communicable germs in the workplace and in schools may reduce lost work productivity and lost school days. The size and shape of the device can be tailored to a particular application, so the device need not unduly add to the bulk of the point-of-contact and can function with many different points of contact. The shapes and materials of the device may increase the efficiency and efficacy of the sources of a sanitizing agent-thereby lowering the necessary power consumption of the system and apparatus-by directing the sanitizing agent toward a location where the point-of-contact may be disposed. Additionally, because the sanitizing device may include one or more of, for example, a switch, timer, or motion sensor, the use and power consumption of the device can be scaled to the frequency that sanitization is necessary for a particular point of contact.

All directional references (e.g., plus, minus, upper, lower, upward, downward, left, right, leftward, rightward, top, bottom, above, below, vertical, horizontal, clockwise, and counterclockwise) are only used for identification purposes to aid the reader's understanding of the present invention. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other.

## Claims

1. A door handle sanitizing apparatus (10) comprising:
a linear portion (16) defining a posterior wall having an inner surface (22);
an arch portion (18) extending in an anterior direction from the posterior wall, the arch portion (18) having an anterior end (34) and an inner surface (22) in common with the inner surface (22) of the linear portion (16), and the arch portion (18) including a concave curve;
a first sanitizing agent source (20₁, 20₂) disposed on the posterior wall inner surface (22); and
a second sanitizing agent source (20₃) disposed on the inner surface (22) of the arch portion (18),
wherein the second sanitizing agent source (20₃) is angled to project the sanitizing agent towards the posterior wall.

2. The door handle sanitizing apparatus of claim 1, wherein the first sanitizing agent source or the second sanitizing agent source is a germicidal ultraviolet light source; and wherein the first sanitizing agent source or the second sanitizing agent source is an LED.

3. The door handle sanitizing apparatus of any preceding claim, wherein the first sanitizing agent source is angled at between 30 degrees and 60 degrees with respect to the posterior wall.

4. The door handle sanitizing apparatus of claim 1, further comprising a motion sensor (48) disposed on the inner surface of the arch portion, wherein the apparatus is configured to deactivate the first and second sanitizing agent sources responsive to the motion sensor detecting motion.

5. The door handle sanitizing apparatus of claim 4, further comprising an electronic controller (54) in electronic communication with the motion sensor, wherein the controller is configured to:
instruct the first and second sanitizing agent sources to begin a sanitization cycle;
receive an indication from the motion sensor that the motion sensor has detected motion;
in response to the indication, instruct the first and second sanitizing agent sources to pause the sanitization cycle; and
instruct the first and second sanitizing agent sources to resume the sanitization cycle.

6. The door handle sanitizing apparatus of claim 5, wherein the controller is further configured to instruct the first and second sanitizing agent sources to resume the sanitization cycle in response to the motion sensor not detecting motion for a predetermined period of time.

7. The door handle sanitizing apparatus of claim 1, wherein:
the linear portion and arched portion share opposed sidewalls,
the inner surface forms an approximately J-shaped surface;
the first sanitizing agent source is a first sanitizing light source, the first sanitizing light source is disposed at a bottom portion on the linear portion and is angled upward so as to project sanitizing light in an anterior direction towards the arch portion; and
the second sanitizing agent source is a second sanitizing light source which is configured to project sanitizing light in a posterior direction towards the linear portion.

8. The door handle sanitizing apparatus of claim 7, wherein the linear portion is configured to be generally parallel with a door when the apparatus is coupled to the door and the first sanitizing light source is configured to project sanitizing light in an anterior direction away from the door.

9. The door handle sanitizing apparatus of claim 8, wherein the second sanitizing light source is configured to project sanitizing light towards the door when the apparatus is coupled with the door.

10. The door handle sanitizing apparatus of claim 7, further comprising:
a third sanitizing light source (20₂) disposed at an upper portion of the linear portion of the inner surface and configured to project sanitizing light towards the arch portion of the inner surface.

11. The door handle sanitizing apparatus of claim 7, further comprising a motion sensor disposed on the arch portion of the inner surface.

12. The door handle sanitizing apparatus of claim 1, wherein:
the linear portion extends from a transition portion to a linear tip; and
the arch portion projects in the anterior direction from the transition portion of the linear portion to an anterior tip;
the inner surfaces of the posterior wall and arch portion are a common inner surface which defines a projected two-dimensional area that is perpendicular to a door and extends along the common inner surface, anterior from the linear tip, and down from the anterior tip;
the apparatus is configured to receive and sanitize a door handle extending through the projected two-dimensional area.

13. The door handle sanitizing apparatus of claim 12, further comprising:
two opposed sidewalls that are generally parallel to each other and generally transverse to the door when the apparatus is affixed to the door;
wherein the common inner surface extends between the two sidewalls.

14. The door handle sanitizing apparatus of claim 12, wherein:
a transverse cross-sectional shape of the common inner surface is a J-shape.

## Patentansprüche

1. Türdrückerdesinfektionsvorrichtung (10), die Folgendes umfasst:
einen linearen Abschnitt (16), der eine Rückwand mit einer Innenoberfläche (22) definiert;
einen Bogenabschnitt (18), der sich von der Rückwand in einer nach vorne gerichteten Richtung erstreckt, wobei der Bogenabschnitt (18) ein vorderes Ende (34) und eine mit der Innenoberfläche (22) des linearen Abschnitts (16) gemeinsame Innenoberfläche (22) aufweist und der Bogenabschnitt (18) eine konkave Krümmung aufweist;
eine erste Desinfektionsmittelquelle (20₁, 20₂), die auf der Rückwandinnenoberfläche (22) platziert ist; und
eine zweite Desinfektionsmittelquelle (20₃), die auf der Innenoberfläche (22) des Bogenabschnitts (18) platziert ist,
wobei die zweite Desinfektionsmittelquelle (20₃) so geneigt ist, dass sie das Desinfektionsmittel zu der Rückwand hin projiziert.

2. Türdrückerdesinfektionsvorrichtung nach Anspruch 1, wobei die erste Desinfektionsmittelquelle oder die zweite Desinfektionsmittelquelle eine Quelle von keimtötendem Ultraviolettlicht ist; und wobei die erste Desinfektionsmittelquelle oder die zweite Desinfektionsmittelquelle eine LED ist.

3. Türdrückerdesinfektionsvorrichtung nach einem der vorhergehenden Ansprüche, wobei die erste Desinfektionsmittelquelle in einem Winkel zwischen 30 Grad und 60 Grad mit Bezug auf die Rückwand geneigt ist.

4. Türdrückerdesinfektionsvorrichtung nach Anspruch 1, die ferner einen auf der Innenoberfläche des Bogenabschnitts platzierten Bewegungssensor (48) umfasst, wobei die Vorrichtung dazu ausgelegt ist, die erste und die zweite Desinfektionsmittelquelle als Reaktion darauf, dass der Bewegungssensor eine Bewegung erkennt, zu deaktivieren.

5. Türdrückerdesinfektionsvorrichtung nach Anspruch 4, die ferner eine elektronische Steuerung (54) in elektronischer Kommunikation mit dem Bewegungssensor umfasst, wobei die Steuerung zu Folgendem ausgelegt ist:
Anweisen der ersten und der zweiten Desinfektionsmittelquelle dazu, mit einem Desinfektionszyklus zu beginnen;
Empfangen einer Anzeige von dem Bewegungssensor, dass der Bewegungssensor eine Bewegung erkannt hat;
als Reaktion auf die Anzeige Anweisen der ersten und der zweiten Desinfektionsmittelquelle dazu, den Desinfektionszyklus zu unterbrechen; und
Anweisen der ersten und der zweiten Desinfektionsmittelquelle dazu, den Desinfektionszyklus fortzusetzen.

6. Türdrückerdesinfektionsvorrichtung nach Anspruch 5, wobei die Steuerung ferner dazu ausgelegt ist, die erste und die zweite Desinfektionsmittelquelle dazu anzuweisen, den Desinfektionszyklus als Reaktion darauf, dass der Bewegungssensor eine vorgegebene Zeit lang keine Bewegung erkennt, fortzusetzen.

7. Türdrückerdesinfektionsvorrichtung nach Anspruch 1, wobei:
der lineare Abschnitt und der Bogenabschnitt gemeinsame, gegenüberliegende Seitenwände aufweisen,
die Innenoberfläche eine näherungsweise J-förmige Oberfläche bildet;
die erste Desinfektionsmittelquelle eine erste Desinfektionslichtquelle ist, die erste Desinfektionslichtquelle an einem unteren Abschnitt auf dem linearen Abschnitt platziert und nach oben geneigt ist, um Desinfektionslicht in einer nach vorne gerichteten Richtung zu dem Bogenabschnitt hin zu projizieren; und
die zweite Desinfektionsmittelquelle eine zweite Desinfektionslichtquelle ist, die dazu ausgelegt ist, Desinfektionslicht in einer nach hinten gerichteten Richtung zu dem linearen Abschnitt hin zu projizieren.

8. Türdrückerdesinfektionsvorrichtung nach Anspruch 7, wobei der lineare Abschnitt so ausgelegt ist, dass er allgemein parallel zu einer Tür ist, wenn die Vorrichtung mit der Tür gekoppelt ist, und die erste Desinfektionslichtquelle dazu ausgelegt ist, Desinfektionslicht in einer nach vorne gerichteten Richtung von der Tür weg zu projizieren.

9. Türdrückerdesinfektionsvorrichtung nach Anspruch 8, wobei die zweite Desinfektionslichtquelle dazu ausgelegt ist, Desinfektionslicht zu der Tür hin zu projizieren, wenn die Vorrichtung mit der Tür gekoppelt ist.

10. Türdrückerdesinfektionsvorrichtung nach Anspruch 7, die ferner Folgendes umfasst:
eine dritte Desinfektionslichtquelle (20₂), die an einem oberen Abschnitt des linearen Abschnitts der Innenoberfläche platziert und dazu ausgelegt ist, Desinfektionslicht zu dem Bogenabschnitt der Innenoberfläche hin zu projizieren.

11. Türdrückerdesinfektionsvorrichtung nach Anspruch 7, die ferner einen auf dem Bogenabschnitt der Innenoberfläche platzierten Bewegungssensor umfasst.

12. Türdrückerdesinfektionsvorrichtung nach Anspruch 1, wobei:
der lineare Abschnitt sich von einem Übergangsabschnitt zu einem linearen Ende erstreckt; und
der Bogenabschnitt in der nach vorne gerichteten Richtung von dem Übergangsabschnitt des linearen Abschnitts zu einem vorderen Ende vorsteht;
es sich bei den Innenoberflächen der Rückwand und des Bogenabschnitts um eine gemeinsame Innenoberfläche handelt, die eine projizierte zweidimensionale Fläche definiert, die senkrecht zu einer Tür verläuft und sich entlang der gemeinsamen Innenoberfläche, von dem linearen Ende nach vorne und von dem vorderen Ende nach unten erstreckt;
die Vorrichtung dazu ausgelegt ist, einen Türdrücker, der sich durch die projizierte zweidimensionale Fläche erstreckt, aufzunehmen und zu desinfizieren.

13. Türdrückerdesinfektionsvorrichtung nach Anspruch 12, die ferner Folgendes umfasst:
zwei gegenüberliegende Seitenwände, die allgemein parallel zueinander und allgemein quer zu der Tür verlaufen, wenn die Vorrichtung an der Tür befestigt ist;
wobei sich die gemeinsame Innenoberfläche zwischen den zwei Seitenwänden erstreckt.

14. Türdrückerdesinfektionsvorrichtung nach Anspruch 12, wobei:
eine querverlaufende Querschnittsform der gemeinsamen Innenoberfläche eine J-Form ist.

## Revendications

1. Appareil de désinfection de poignée de porte (10) comprenant :
une partie linéaire (16) définissant une paroi postérieure ayant une surface interne (22) ;
une partie arquée (18) s'étendant dans une direction antérieure à partir de la paroi postérieure, la partie arquée (18) ayant une extrémité antérieure (34) et une surface interne (22) commune avec la surface interne (22) de la partie linéaire (16), et la partie arquée (18) incluant une courbe concave ;
une première source d'agent désinfectant (20₁, 20₂) disposée sur la surface interne de la paroi postérieure (22) ; et
une deuxième source d'agent désinfectant (20₃) disposée sur la surface interne (22) de la partie arquée (18),
dans lequel la deuxième source d'agent désinfectant (20₃) est inclinée afin de projeter ce dernier vers la paroi postérieure.

2. Appareil de désinfection de poignée de porte selon la revendication 1, dans lequel la première ou la deuxième source d'agent désinfectant est une source de lumière ultraviolette germicide ; et dans lequel la première source ou la deuxième source d'agent désinfectant est une LED.

3. Appareil de désinfection de poignée de porte selon l'une quelconque des revendications précédentes, dans lequel la première source d'agent désinfectant est inclinée selon un angle entre 30 degrés et 60 degrés par rapport à la paroi postérieure.

4. Appareil de désinfection de poignée de porte selon la revendication 1, comprenant en outre un capteur de mouvement (48) disposé sur la surface interne de la partie arquée, l'appareil étant configuré pour désactiver les première et deuxième sources d'agent désinfectant en réponse à la détection d'un mouvement par le capteur de mouvement.

5. Appareil de désinfection de poignée de porte selon la revendication 4, comprenant en outre un dispositif de commande électronique (54) en communication électronique avec le capteur de mouvement, dans lequel le dispositif de commande est configuré pour :
commander aux première et deuxième sources d'agent désinfectant de commencer un cycle de désinfection ;
recevoir une indication en provenance du capteur de mouvement lorsque celui-ci a détecté un mouvement ;
en réponse à l'indication, commander aux première et deuxième sources d'agent désinfectant de mettre en pause le cycle de désinfection ; et
commander aux première et deuxième sources d'agent désinfectant de reprendre le cycle de désinfection.

6. Appareil de désinfection de poignée de porte selon la revendication 5, dans lequel le dispositif de commande est en outre configuré pour commander aux première et deuxième sources d'agent de désinfection de reprendre le cycle de désinfection en réponse à la non-détection d'un mouvement par le capteur de mouvement sur une période prédéterminée.

7. Appareil de désinfection de poignée de porte selon la revendication 1, dans lequel :
la partie linéaire et la partie arquée partagent de parois latérales opposées,
la surface interne est à peu près en forme de J ;
la première source d'agent désinfectant est une première source de lumière de désinfection disposée dans une partie inférieure de la partie linéaire inclinée vers le haut de façon à projeter une lumière de désinfection dans une direction antérieure vers la partie arquée ; et
la deuxième source d'agent désinfectant est une deuxième source de lumière de désinfection configurée pour projeter une lumière de désinfection dans une direction postérieure vers la partie linéaire.

8. Appareil de désinfection de poignée de porte selon la revendication 7, dans lequel la partie linéaire est configurée pour être généralement parallèle à une porte lorsque l'appareil est couplé à celle-ci et la première source de lumière de désinfection est configurée pour projeter une lumière de désinfection dans une direction antérieure à l'opposé de la porte.

9. Appareil de désinfection de poignée de porte selon la revendication 8, dans lequel la deuxième source de lumière de désinfection est configurée pour projeter une lumière de désinfection dans une direction postérieure vers la porte lorsque l'appareil est couplé à celle-ci.

10. Appareil de désinfection de poignée de porte selon la revendication 7, comprenant en outre :
une troisième source de lumière de désinfection (20₂) disposée au niveau d'une partie supérieure de la partie linéaire de la surface interne et configurée pour projeter une lumière de désinfection vers la partie arquée de la surface interne.

11. Appareil de désinfection de poignée de porte selon la revendication 7, comprenant en outre un capteur de mouvement disposé sur la partie arquée de la surface interne.

12. Appareil de désinfection de poignée de porte selon la revendication 1, dans lequel :
la partie linéaire s'étend d'une partie de transition vers une pointe linéaire ; et
la partie arquée fait saillie dans une direction antérieure depuis la partie de transition de la partie linéaire vers une pointe antérieure ;
les surfaces internes de la paroi postérieure et de la partie arquée sont une surface interne commune définissant une zone bidimensionnelle en saillie perpendiculaire à une porte et s'étendant le long de la surface interne, de manière antérieure depuis la pointe linéaire, et vers le bas depuis la pointe antérieure ;
l'appareil est configuré pour recevoir et désinfecter une poignée de porte s'étendant à travers la zone bidimensionnelle en saillie.

13. Appareil de désinfection de poignée de porte selon la revendication 12, comprenant en outre :
deux parois latérales opposées généralement parallèles l'une à l'autre et généralement transversales par rapport à la porte lorsque l'appareil est fixé sur celle-ci ;
dans lequel la surface intérieure commune s'étend entre les deux parois latérales.

14. Appareil de désinfection de poignée de porte selon la revendication 12, dans lequel :
une forme de section transversale de la surface interne commune est en J.
